(19) Europäisches Patentamt
European Patent Office
Office européen des brevets

(11) Numéro de publication: **0 078 757**
**B1**

(12) **FASCICULE DE BREVET EUROPEEN**

(45) Date de publication du fascicule du brevet: **04.09.85**

(21) Numéro de dépôt: **82450015.1**

(22) Date de dépôt: **27.10.82**

(51) Int. Cl.⁴: **C 07 D 295/12**, C 07 D 213/53, C 07 D 333/22, C 07 D 307/52, C 07 D 317/66, A 61 K 31/495

(54) **Nouvelles imines dérivées de (phényl-4 pipérazinyléthyl)-2 anilines, leur méthode de préparation, ainsi que leur emploi en thérapeutique.**

(30) Priorité: **03.11.81 FR 8120563**

(43) Date de publication de la demande: **11.05.83 Bulletin 83/19**

(45) Mention de la délivrance du brevet: **04.09.85 Bulletin 85/36**

(84) Etats contractants désignés: **BE CH DE GB IT LI LU NL**

(56) Documents cités: **FR - A - 2 476 644**

(73) Titulaire: **SOCIETE CORTIAL S.A., 7 rue de l'Armorique, F-75015 Paris (FR)**

(72) Inventeur: **Pontagnier, Henri, 21 rue Edouard Vaillant, F-33600 Pessac (FR)**
Inventeur: **Creuzet, Marie-Hélène, Résidence Jardin de Gambetta T3, F-33000 Bordeaux (FR)**
Inventeur: **Guichard, Françoise, Les Cèdres Parc des Tourelles rue Ste Elisabeth, F-33200 Bordeaux (FR)**
Inventeur: **Prat, Gisèle, Hameau de Noailles Villa 18, F-33400 Talence (FR)**

(74) Mandataire: **Tajan, Marie-Thérèse, LABORATOIRES SARGET Avenue du Président JF Kennedy, F-33701 Mérignac (FR)**

**EP 0 078 757 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouvelles imines dérivées de (phényl-4 pipérazinyléthyl)-2 anilines, leur méthode de préparation ainsi que leur emploi en thérapeutique.

Ces nouveaux produits ont pour formule générale

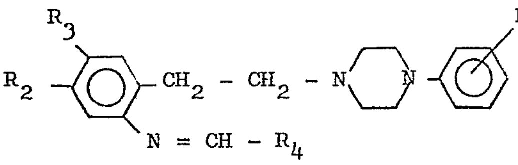 (I)

dans laquelle $R_1$ représente H ou un ou plusieurs substituants situés en ortho, méta ou para choisis parmi $CH_3$, $CF_3$, Cl, $OCH_3$ et F;

$R_2$, $R_3$ identiques ou différents sont H ou alcoxy inférieur de C1 à C4, $R_2$ et $R_3$ pouvant constituer ensemble une chaîne $-O-(CH_2)_n-O-$ avec n=1 ou 2 ou une chaîne $-O-CH_2-O-CH_2-$;

$R_4$ représente phényle ou phényle substitué par un ou plusieurs substituants choisis parmi halogéno, alkyle de C1 à C4, alcoxy de C1 à C4 et trifluorométhyle, ou $R_4$ est furyle ou thiényle substitué par halogéno ou $CH_3$ ou $R_4$ est pyridinyl-2, -3 ou -4.

Ces produits peuvent être sous forme de base libre ou de sels pharmaceutiquement compatibles tels que chlorhydrates, citrates, benzilates.

On connaissait déjà des imines dérivées de la (méthyl-4 pipérazinyléthyl)-2 aniline (brevet français N° 2476644). Or alors que ces dernières présentent des propriétés psychotropes du type anxiolytique ou antidépresseur nous venons de découvrir que les dérivés de phényl-4 pipérazine faisant l'objet de la présente invention présentent des propriétés psychotropes totalement différentes se caractérisant par des effets neuroleptiques du type sédatif et peu cataleptigène. Ces produits présentent également des activités antianaphylactiques, antihistaminiques et antisérotoninergiques. Ces activités jointes à une faible toxicité permettent l'emploi des produits de la présente invention par exemple en thérapeutique psychotrope et en allergologie.

Les produits de la présente invention sont obtenus de façon générale par une réaction de condensation entre une amine de formule

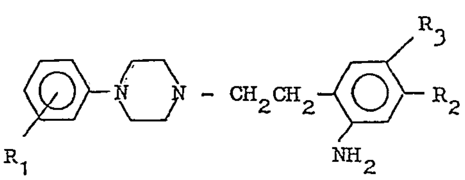 (II)

dont la préparation est décrite et un aldéhyde de formule

$$R_4-CHO \qquad (III),$$

en présence d'un solvant tel que le benzène. L'eau formée au cours de la réaction est éliminée à l'aide d'un piège de Dean-Stark et le résidu est recristallisé dans un solvant tel qu'un mélange éther éthylique, éther de pétrole.

*Exemple 1 :*

*Préparation de la (phényl-4 pipérazinyléthyl)-2 N-pyridylidène-3 aniline* (produit de formule I avec $R_1$=H, $R_2$=$R_3$=H, $R_4$= 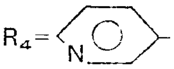).

On chauffe sous agitation à 140° C dans un ballon à fond rond de 500 cm³ le mélange constitué de 0,035 mole de (phényl-4 pipérazinyléthyl)-2 aniline, 0,070 mole de nicotinaldéhyde, 60 cm³ de benzène anhydre. L'eau formée au cours de la réaction est éliminée à l'aide d'un piège de Dean-Stark. Le mélange est ainsi chauffé sous agitation jusqu'à ce que l'on ait recueilli 0,035 mole d'eau. L'excès d'aldéhyde est éliminé par distillation. Le résidu de distillation est dissous dans le minimum d'éther éthylique. De l'éther de pétrole est rajouté et le mélange est refroidi à −30° C. Le précipité est filtré. On obtient ainsi l'imine de point de fusion 65° C.

*Exemple 2:*

On prépare selon le procédé décrit dans l'exemple 1 les produits de formule I tels que $R_2$=$R_3$=H et

$R_1$=$CF_3$−3 $R_4$= 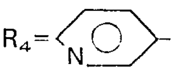; produit huileux

$R_1$=F−4 $R_4$= 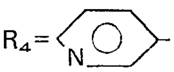; PF=95° C

$R_1$=$OCH_3$−2 $R_4$= 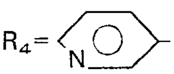; PF=95° C

$R_1$=$CH_3$−2 $R_4$= 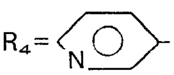; produit huileux

$R_1$=$CF_3$−3 $R_4$=phényl; produit huileux
$R_1$=F−4 $R_4$=phényl; PF=90° C

$R_1$=H $R_4$=$CH_3$− 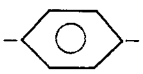; PF=70° C

$R_1$=$OCH_3$−2 $R_4$=$CH_3$− 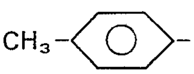; produit huileux

$R_1$=F−4 $R_4$= 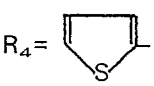; PF=88° C

$R_1$=Cl−4 $R_4$= 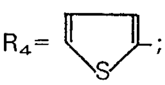; PF=102° C

$R_1$=$CF_3$−3 $R_4$= 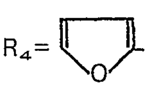; produit huileux

$R_1$=$CH_3$−2 $R_4$= 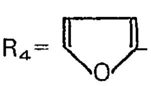; PF=94° C

$R_1$=F−4 $R_4$=(triméthoxy-3,4,5 phényl); PF=95° C

$R_1$=Cl−4 $R_4$=(triméthoxy-3,4,5 phényl); PF=80° C

$R_1$=Cl−4 $R_4$= 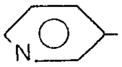; PF=130° C

$R_1$=$CH_3$−2 $R_4$=$CH_3$− 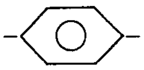; produit huileux

*Exemple 3:*

*Préparation du citrate du produit de l'exemple 1* ou COR 34 12.

0,025 mole du produit de l'exemple 1 est dissoute dans 1500 $cm^3$ d'éther éthylique anhydre. Cette solution est refroidie entre −20 et −30° C. 0,025 mole d'acide citrique dissoute dans le minimum de méthanol est ajoutée goutte à goutte sous agitation. Le précipité est filtré rapidement et séché dans un dessiccateur en présence de $P_2O_5$ sous le vide de la pompe à palettes. On obtient ainsi un produit hygroscopique de masse moléculaire 562.

La masse moléculaire exacte des citrates est déterminée par un dosage potentiométrique de l'acide citrique.

*Exemple 4:*

On prépare selon le procédé décrit dans l'exemple 3 les citrates des produits de formule I tels que $R_2$=$R_3$=H et

$R_1$=$CF_3$−3 $R_4$= 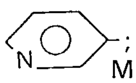; COR 3422; M(masse moléculaire expérimentale)=701,6; produit hygroscopique

$R_1$=F−4 $R_4$= 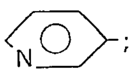; COR 3418; M=632,5; produit hygroscopique

$R_1$=$OCH_3$−2 $R_4$= 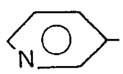; COR 3419; M=636,8; produit hygroscopique

$R_1$=Cl−4 $R_4$= 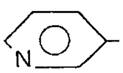; COR 3425; M=597,0; produit hygroscopique

$R_1$=$CH_3$−2 $R_4$= 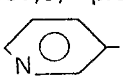; COR 3426; M=597,8; produit hygroscopique

$R_1$=$CF_3$−3 $R_4$= 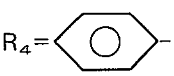; COR 3431; M=629,6; F=134° C

$R_1$=F−4 $R_4$= 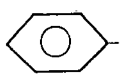; COR 3424; M=604,6; produit hygroscopique

$R_1$=H $R_4$=Me− 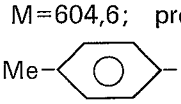; COR 3421; M=575,6; produit hygroscopique

$R_1$=$OCH_3$−2 $R_4$=Me− 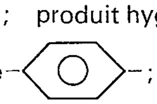; COR 3420; M=605,7; produit hygroscopique

$R_1$=$CH_3$−2 $R_4$=Me− 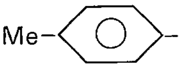; COR 3427; M=628,1; produit hygroscopique

$R_1$=F−4 $R_4$= 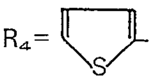; COR 3436; M=593,3; F=150° C

$R_1$=Cl−4 $R_4$= 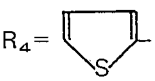; COR 3433; M=602,1; F=145° C

$R_1$=$CF_3$−3 $R_4$= 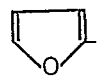; COR 3432; M=636,9; F=120° C

$R_1$=$CH_3$−2 $R_4$= 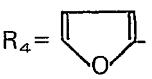; COR 3434; M=565,6; produit hygroscopique

$R_1$=F−4 $R_4$= 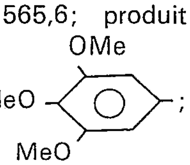; COR 3435; M=669,7; produit hygroscopique

$R_1$=Cl−4 $R_4$= 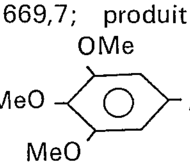; COR 3429; M=686,1; produit hygroscopique

*Exemple 5:*

*Synthèse de la (((trifluorométhyl-3 phényl)-4 pipérazinyl)-2 éthyl)-6 N-pyridylidène-3 benzodioxol-1,3 yl-5 amine* (produit de formule I avec $R_1$=$CF_3$−3, $R_2$−$R_3$= −O−$CH_2$−O−; $R_4$= 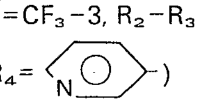)

On chauffe à 120° C le mélange composé de 8 g de (((trifluorométhyl-3 phényl)-4 pipérazinyl)-2 éthyl)-6 benzodioxol-1,3 yl-5 amine, 20 g de nicotinaldéhyde et 300 $cm^3$ de benzène.

L'eau formée au cours de la réaction est éliminée à l'aide d'un piège de Dean-Stark. Le benzène est éliminé par évaporation. L'excès d'aldéhyde est distillé. Le résidu est recristallisé dans un mélange d'éther de pétrole et d'éther éthylique. Le produit de l'exemple 5 est obtenu avec un rendement de 84%. F=100° C.

*Exemple 6:*

*Préparation du citrate du produit de l'exemple 5 ou COR 3415*

Deux solutions sont préparées; l'une contient 5,14 g du produit de l'exemple 5 dans le minimum de méthanol, l'autre contient 2,03 g d'acide citrique dissous dans le minimum de méthanol. Ces deux solutions sont mélangées et ce mélange est

ajouté goutte à goutte dans deux litres d'éther éthylique anhydre refroidi à −5° C sous agitation. Après filtration, le produit est séché sous le vide de la pompe à 80° C. Masse expérimentale: 722,5.

Les produits de la présente invention ont fait l'objet de contrôles physico-chimiques (microanalyse, RMN) dont les résultats sont donnés ci-après.

*Microanalyse*

| Produit | Valeurs théoriques | | | | Valeurs expérimentales | | | |
|---|---|---|---|---|---|---|---|---|
| | C | H | O | N | C | H | O | N |
| COR3412 | 64,04 | 6,09 | 19,91 | 9,96 | 64,01 | 6,48 | 19,72 | 9,70 |
| COR3415 | 55,68 | 4,88 | | 7,75 | 55,21 | 4,85 | | 7,68 |
| COR3418 | 60,05 | 5,60 | 22,49 | 8,86 | 60,15 | 5,85 | 22,15 | 8,94 |
| COR3419 | 61,07 | 5,99 | 24,15 | 8,80 | 61,03 | 6,42 | 24,21 | 9,03 |
| COR3420 | 65,44 | 6,49 | 21,13 | 6,94 | 65,24 | 6,77 | 21,12 | 7,20 |
| COR3421 | 66,77 | 6,48 | 19,46 | 7,30 | 66,76 | 6,76 | 19,32 | 7,45 |
| COR3422 | 56,86 | 5,17 | 21,87 | 7,98 | 56,85 | 5,52 | 22,41 | 8,03 |
| COR3424 | 63,13 | 5,84 | 20,93 | 6,95 | 62,99 | 6,06 | 20,15 | 7,57 |
| COR3425 | 60,35 | 5,57 | | 9,38 | 60,54 | 6,34 | | 9,19 |
| COR3426 | 63,70 | 6,23 | 20,68 | 9,40 | 63,71 | 6,32 | 20,33 | 9,51 |
| COR3427 | 65,40 | 6,52 | 21,40 | 6,69 | 65,67 | 6,98 | 20,93 | 6,96 |
| COR3429 | 59,51 | 5,87 | 23,31 | 6,12 | 59,21 | 5,87 | 23,80 | 5,86 |
| | | | Cl 5,16 | | | | Cl 5,29 | |
| COR3431 | 61,04 | 5,44 | | 6,67 | 60,82 | 5,51 | | 6,61 |
| | | | F 9,05 | | | | F 9,61 | |
| COR3432 | 57,59 | 5,18 | | 6,60 | 57,62 | 5,06 | | 6,73 |
| COR3433 | 57,85 | 5,35 | 18,60 | 6,97 | 57,77 | 5,32 | 18,64 | 6,86 |
| | | | Cl 5,88 | | | | Cl 5,77 | |
| | | | S 5,32 | | | | S 5,26 | |
| COR3434 | 63,70 | 6,24 | 22,63 | 7,43 | 63,74 | 6,60 | 22,68 | 7,42 |
| COR3435 | 60,98 | 6,02 | | 6,27 | 60,87 | 5,91 | | 6,27 |
| | | | F 2,84 | | | | F 2,99 | |
| COR3436 | 59,19 | 5,49 | | 7,08 | 59,38 | 5,49 | | 7,22 |
| | | | F 3,20 | | | | F 3,22 | |
| | | | S 5,40 | | | | S 5,35 | |

Le tableau des résultats RMN contient les principales caractéristiques des spectres de certains produits de la présente invention mis en solution dans DMSO D6. Sont indiqués les déplacements chimiques par rapport au TMS pris comme étalon interne, le nombre de protons et la multiplicité des pics avec mc=massif complexe; s=singulet; d=dôme.

*(Tableau en page suivante)*

Les produits faisant l'objet de la présente invention ont subi divers tests pharmacologiques dont nous donnons certains résultats ci-après.

La mortalité induite par ces substances a été déterminée chez la souris Swiss exempte d'organismes pathogènes spécifiques. Les animaux sont stabulés en salle climatisée 24 à 48 heures avant le début de l'expérimentation. Les substances sont administrées soit par voie intragastrique en solution dans de l'huile d'olive ou du tween, soit par voie intrapéritonéale en solution dans du tween. Les résultats sont exprimés dans les tableaux I et II par le pourcentage de mortalité en fonction de la dose administrée ou par la dose induisant 50% de mortalité (DL 50) déterminée par la méthode de Bliss.

L'activité antihistaminique a été déterminée in vitro par la mesure de la concentration entraînant 50% d'inhibition des contractions induites par l'histamine ($1 \cdot 10^{-8}$ g/l) sur iléon de cobaye (CI 50). Elle est représentée dans le tableau III.

Les produits de la présente invention possèdent une activité antisérotonine in vitro. Nous donnons dans le tableau N° IV les doses de substance entraînant in vitro plus de 80% d'inhibition de la sérotonine.

L'activité antiallergique a été déterminée dans le test de l'anaphylaxie passive cutanée de la façon suivante. On administre à des rats mâles Sprague Dawley par voie intradermique 0,1 ml de sérum riche en IgE. Ce sérum a été fabriqué en sensibilisant des rats Sprague-Dawley par une injection ip d'ovalbumine et de suspension de Bordetella pertusis suivie 20 j après par une nouvelle injection d'ovalbumine. 24 heures après administration du sérum on injecte par voie iv 0,5 ml d'une solution contenant 8,25 mg/kg d'ovalbumine et 26,4 mg/kg de bleu d'Evans dans un tampon pH 7,05. Au bout de 30 min les animaux sont sacrifiés; chaque papule est découpée, pesée et mise à incuber pendant 4 j à 37° C dans 15 g de formamide. La quantité de bleu d'Evans contenue dans chaque papule est déterminée par la mesure de la densité

| Code | $CH_2-C-CH_2$ (Acide citrique) | 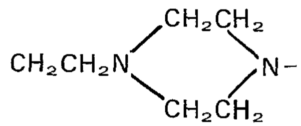 | Protons aromatiques | N=CH | 40H Acide citrique | Divers |
|---|---|---|---|---|---|---|
| COR3412 | 2,7ppm; 4H; s | 2,8-3,6ppm; 12H; mc | 6,6-9,1ppm; 14H; mc (8,6ppm; 1H; S) | | 9,9ppm; 4H; pic large | |
| COR3415 | 2,7ppm; 4H; s | 2,9-3,8ppm; 12H; mc | 6,9-9,1ppm; 11H; mc (8,7ppm; 1H; s) | | 9,5ppm; 4H; pic large | 6,0ppm; 2H; $OCH_2O$ |
| COR3418 | 2,7ppm; 4H; s | 2,8-3,5ppm; 12H; mc | 6,8-9,1ppm; 13H; mc (8,6ppm; 1H; s) | | 10,2ppm; 4H; pic large | |
| COR3419 | 2,7ppm; 4H; s | 2,8-3,5ppm; 12H; mc | 6,7-9,1ppm; 13H; mc (8,6ppm; 1H; s) | | 10,2ppm; 4H; pic large | 3,8ppm; 3H; s; $OCH_3$ |
| COR3420 | 2,7ppm; 4H; s | 2,8-3,5ppm; 12H; mc | 6,7-8,0ppm; 12H; mc | 8,5ppm; 1H; s | 10,1ppm; 4H; pic large | 2,4ppm; 3H; s; $CH_3C$ 3,8ppm; 3H; s; $OCH_3$ |
| COR3421 | 2,7ppm; 4H; s | 2,8-3,5ppm; 12H; mc | 6,6-8,0ppm; 13H; mc | 8,5ppm; 1H; s | 10,0ppm; 4H; pic large | 2,4ppm; 3H; s; $OCH_3$ |
| COR3422 | 2,7ppm; 4H; s | 2,9-3,7ppm; 12H; mc | 6,9-9,1ppm; 13H; mc (8,6ppm; 1H; s) | | 10,4ppm; 4H; pic large | |
| COR3424 | 2,7ppm; 4H; s | 2,8-3,5ppm; 12H; mc | 6,8-8,2ppm; 13H; mc | 8,5ppm; 1H; s | 10,2ppm; 4H; pic large | |
| COR3425 | 2,7ppm; 4H; s | 2,8-3,6ppm; 12H; mc | 6,8-9,2ppm; 13H; mc (8,7ppm; 1H; s) | | 10,4ppm; 4H; pic large | |
| COR3426 | 2,7ppm; 4H; s | 2,8-3,5ppm; 12H; mc | 6,8-9,1ppm; 13H; mc (8,6ppm; 1H; s) | | 9,7ppm; 4H; pic large | 2,2ppm; 3H; s; $CH_3C$ |
| COR3427 | 2,6ppm; 4H; s | 2,8-3,4ppm; 12H; mc | 6,9-8,0ppm; 12H; mc | 8,5ppm; 1H; s | 8,9ppm; 4H; pic large | 2,2ppm; 3H; s; $CH_3o$ 2,4ppm; 3H; s; $CH_3p$ |
| COR3429 | 2,6ppm; 4H; s | 2,7-3,5ppm; 12H; mc | 6,7-7,5ppm; 10H; mc | 8,4ppm; 1H; s | 8,8ppm; 4H; d | 3,7 et 3,8ppm; 9H; 2s; $3CH_3O$ |
| COR3431 | 2,7ppm; 4H; s | 2,7-3,6ppm; 12H; mc | 6,9-8,2ppm; 13H; mc | 8,5ppm; 1H; s | 9,4ppm; 4H; pic large | |
| COR3432 | 2,7ppm; 4H; s | 2,8-3,6ppm; 12H; mc | 6,6-8,0ppm; 11H; mc | 8,3ppm; 1H; s | 8,4ppm; 4H; pic large | |
| COR3433 | 2,6ppm; 4H; s | 2,7-3,4ppm; 12H; mc | 6,7-7,8ppm; 11H; mc | 8,6ppm; 1H; s | 9,4ppm; 4H; pic large | |
| COR3434 | 2,6ppm; 4H; s | 2,7-3,2ppm; 12H; mc | 6,6-7,9ppm; 11H; mc | 8,3ppm; 1H; s | 9,2ppm; 4H; pic large | 2,2ppm; 3H; s; $CH_3C$ |
| COR3435 | 2,6ppm; 4H; s | 2,7-3,4ppm; 12H; mc | 6,7-7,4ppm; 10H; mc | 8,4ppm; 1H; s | 9,1ppm; 4H; d | 3,7 et 3,8ppm; 9H; s; 3 $OCH_3$ |
| COR3436 | 2,7ppm; 4H; s | 2,8-3,4ppm; 12H; mc | 6,8-7,9ppm; 11H; mc | 8,7ppm; 1H; s | 9,6ppm; 4H; pic large | |

optique du mélange. Le produit à tester est administré par voie orale ou intrapéritonéale en suspension homogène en présence de tween 80, 10 min avant l'injection déclenchante. Son activité est appréciée par le pourcentage de diminution de la quantité de bleu d'Evans ayant diffusé dans la papule. Les DE 50 de certains produits de la présente invention sont indiquées dans le tableau V.

*TABLEAU I*

*Toxicité par voie orale*

| Produit COR N° | Solvant | % de mortalité en fonction de la dose exprimée en mg/kg | | | | | | DL 50 en mg/kg |
|---|---|---|---|---|---|---|---|---|
| | | 300 | 500 | 750 | 1000 | 1500 | 2000 | |
| 3412 | huile olive | | | | 0- 10 | 0 | | |
| 3415 | huile olive | | 0 | 50 | 60 | | 70 | |
| 3418 | tween 20% | | 0 | 20 | 30 | | | |
| 3419 | tween 20% | 20 | 75 | | | | | 427 (384-474) |
| 3420 | tween 20% | | 60 | 75 | 90 | | | 523 (440-621) |
| 3421 | tween 20% | | 0 | 30 | 100 | | | 792 (746-839) |
| 3422 | tween 20% | 0 | | | | | | |
| 3425 | tween 20% | | 20 | 90 | 100 | | | 584 (536-637) |
| 3426 | tween 20% | | 20 | 50 | 100 | | | 676 (578-791) |
| 3427 | tween 20% | | 10 | 70 | 80 | | | 733 (623-863) |
| 3429 | tween 20% | | 10 | 70 | 80 | | | 666 (580-765) |
| 3431 | tween 10 ou 20% | | 0 | 0 | 30 | | | |
| 3432 | tween 10 ou 20% | | 0 | 20 | 50 | | | |
| 3433 | tween 10 ou 20% | | 0 | | 0 | | | |
| 3434 | tween 20% | | 50 | 70 | 100 | | | 562 (489-644) |
| 3435 | tween 20% | | 0 | 30 | 90 | | | 864 (807-926) |
| 3436 | tween 10 ou 20% | | 0 | | 0 | | | |

*TABLEAU II*

Toxicité par voie intrapéritonéale

| Produit COR N° | DL 50 en mg/kg |
|---|---|
| 3421 | 254 (236-274) |
| 3424 | 288 (260-318) |
| 3425 | 187 (165-212) |
| 3426 | 185 (166-207) |
| 3427 | 330 (292-373) |

*TABLEAU III* →

Activité antihistaminique in vitro

| Produit COR N° | CI 50 en M/l |
|---|---|
| 3412 | 4,88 (2,16 -10,98)$\cdot 10^{-7}$ |
| 3415 | 3,23 (2,25 - 4,61)$\cdot 10^{-6}$* |
| 3418 | 7,62 (5,33 -10,91)$\cdot 10^{-7}$ |
| 3419 | 3,27 (1,95 - 5,46)$\cdot 10^{-6}$* |
| 3420 | 2,51 (1,21 - 5,17)$\cdot 10^{-6}$ |
| 3421 | 2,81 (1,09 - 7,28)$\cdot 10^{-6}$ |
| 3422 | 1,21 (0,742- 1,98)$\cdot 10^{-5}$ |
| 3424 | 1,98 (1,16 - 3,43)$\cdot 10^{-6}$ |
| 3425 | 3,57 (2,28 - 5,59)$\cdot 10^{-6}$* |
| 3426 | 4,68 (3,64 - 5,98)$\cdot 10^{-6}$* |
| 3427 | 8,72 (5,05 -15,0 )$\cdot 10^{-6}$* |
| 3429 | 6,08 (3,0 -12,3 )$\cdot 10^{-6}$* |
| 3431 | 1,20 (0,543- 2,70)$\cdot 10^{-5}$* |
| 3432 | 7,76 (4,73 -12,7 )$\cdot 10^{-6}$* |
| 3433 | 3,59 (2,52 - 5,10)$\cdot 10^{-6}$* |
| 3434 | 7,02 (5,39 - 9,16)$\cdot 10^{-6}$* |
| 3435 | 5,90 (4,61 - 7,54)$\cdot 10^{-6}$* |
| 3436 | 2,36 (1,11 - 5,02)$\cdot 10^{-6}$ |
| Prométhazine | 1,27 (0,761- 2,12)$\cdot 10^{-7}$* |
| Chlorpromazine | 3,63 (2,18 - 4,20)$\cdot 10^{-6}$* |

* Activité mesurée après rinçage.

*TABLEAU IV*

Activité antisérotonine

| Produit N° | Dose active exprimée en g/ml |
|---|---|
| 3412 | $10^{-4}$ |
| 3418 | $5\cdot10^{-5}$ |
| 3422 | $2\cdot10^{-6}$ |
| 3424 | $2,5\cdot10^{5}$ |
| 3425 | $4\cdot10^{-7}$ |
| 3427 | $2,5\cdot10^{-5}$ |
| Cyproheptadine | $10^{-6}$ |
| Miansérine | $10^{-5}$ |

*TABLEAU V*

Test de l'anaphylaxie passive cutanée

| Produit N° | DE 50 (mg/kg) voie po | DE 50 (mg/kg) voie ip |
|---|---|---|
| COR 3412 | 11,9 ( 7,9 -18,2 ) | 7,52 (3,31-17,38) |
| COR 3415 | 20 | |
| COR 3418 | 5,7 ( 3,7 - 8,5 ) | |
| COR 3419 | 13,3 ( 9,3 -18,6 ) | 5,65 (3,24- 9,77) |
| COR 3420 | 7,12 ( 4,37-11,48) | 3,85 (2,95- 5,13) |
| COR 3421 | 8,0 ( 5,62-11,22) | 6,79 (4,79- 9,55) |
| COR 3424 | 9 | |
| COR 3425 | 5 | |
| COR 3426 | 3,3 ( 1,35- 8,13) | |
| COR 3427 | 4,5 ( 3,2 - 6,3 ) | |
| COR 3429 | 8,76 ( 4,07-18,62) | |
| COR 3431 | 27,5 (20,8 -36,3 ) | |
| COR 3432 | 43 (32,3 -56,2 ) | |
| COR 3433 | 17,09 ( 9,77-29,51) | |
| COR 3434 | 5,34 ( 3,31- 8,71) | |
| COR 3435 | 7,82 ( 3,89-15,49) | |
| COR 3436 | 6,29 ( 4,79- 8,31) | |
| Chlorpromazine | 4,9 ( 2,1 -11,2 ) | |
| Prométhazine | 2,9 ( 1,9 - 4,3 ) | |

L'activité psychotrope des produits faisant l'objet de la présente invention est caractérisée par un profil du type neuroleptique avec diminution de l'activité motrice, antagonisme de certains effets de l'amphétamine et de l'apomorphine. L'effet cataleptigène apparaît à des doses élevées.

L'effet des substances sur la motilité est appréciée chez la souris en utilisant un actimètre. Les animaux sont placés dans la cage d'activométrie et à partir du nombre de déplacements enregistrés par l'appareil, on obtient pour chaque dose considérée le pourcentage de l'effet observé par rapport au lot témoin. Ces pourcentages sont reportés sur un papier à l'échelle log-probit et la méthode de Litchfield Wilcoxon permet de calculer la dose efficace 50 sédative. Le tableau VI donne les DE 50 sédatives pour un certain nombre de produits faisant l'objet de l'invention.

*TABLEAU VI*

| Produit N° | DE 50 sédation en mg/kg voie ip |
|---|---|
| COR 3412 | 4 |
| COR 3418 | 4 |
| COR 3419 | 9 |
| COR 3420 | 14 |
| COR 3421 | 7 |
| COR 3422 | 20 |
| COR 3424 | 6 |
| COR 3425 | 12 |
| COR 3426 | 7 |
| COR 3427 | 12 |

L'effet cataleptigène est recherché chez le rat. Après l'administration intrapéritonéale du produit à tester, chaque rat est observé 30 min, 1 h, 2 h, 4 h et 6 h après l'injection et subit à ces échéances de temps les tests du Bouddha et du croisement homolatéral des pattes. On dispose de lots de 6 animaux par dose de produit. Les substances faisant l'objet de la présente invention sont cataleptigènes à des doses voisines des doses létales (128-256 mg/kg). Les produits de la présente invention antagonisent les redressements et les stéréotypies induits par l'apomorphine chez la souris. Par contre ils n'inhibent pas l'hypothermie induite par l'apomorphine. Les produits de l'invention inhibent la toxicité de groupe induite par l'amphétamine à des doses comprises entre 25 et 100 mg/kg par voie ip.

Compte tenu de leurs propriétés antihistaminiques, antisérotoninergiques et antianaphylactiques jointes à une faible toxicité, les produits de la présente invention sont utiles en thérapeutique employés seuls ou en association dans le traitement des états allergiques et anaphylactiques tels que l'urticaire, les prurits, les dermatoses, les eczémas, le rhume de foins, l'œdème de Quincke, la maladie sérique, l'asthme, le choc anaphylactique. Ils peuvent être employés à titre préventif ou curatif dans le traitement du mal des transports. Leurs propriétés psychotropes permettent de les employer seuls ou en association dans le traitement des états névrotiques et psychotiques, des états d'agitation et d'excitation, des manifestations d'agressivité. Ils pourront être administrés par voie orale sous forme par exemple de dragées, comprimés, sirop, ampoules, par voie rectale sous forme de suppositoire, par voie intramusculaire ou intraveineuse ou par voie topique sous forme de pommade ou gel. Les doses administrées varieront selon l'indication et le sujet de 1 à 100 mg/j en 2 à 6 prises pour la voie orale, de 1 à 100 mg/j en 1 ou 2 prises pour la voie rectale, de 0,5 à 50 mg par injection pour les voies parentérales.

## Revendications

1. Produits correspondant à la formule générale

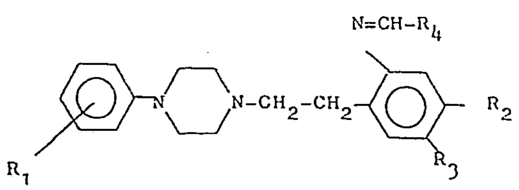

ainsi que leurs sels d'addition non toxiques, dans laquelle $R_1$ représente H ou un ou plusieurs substituants situés en ortho, méta ou para choisis parmi $CH_3$, $CF_3$, Cl, $OCH_3$ et F; $R_2$, $R_3$ identiques ou différents sont H ou alcoxy inférieur de C1 à C4, $R_2$ et $R_3$ pouvant constituer ensemble une chaîne $-O-(CH_2)_n-O-$ avec n=1 ou 2 ou une chaîne $-O-CH_2-O-CH_2-$; $R_4$ représente phényl ou phényle substitué par un ou plusieurs substituants choisis parmi halogéno, alkyle de C1 à C4, alcoxy de C1 à C4 et trifluorométhyle ou $R_4$ est furyle ou thiényle substitué par halogène ou $CH_3$ ou $R_4$ est pyridinyl-2,-3 ou -4.

2. Produits selon la revendication 1, caractérisés en ce que $R_2=R_3=H$.

3. Produits selon la revendication 1, caractérisés en ce que $R_2$ et $R_3$ forment ensemble une chaîne $-O-CH_2-O-$.

4. Procédé de préparation des produits selon les revendications 1 à 3, caractérisé en ce que l'on chauffe le mélange constitué par une amine de formule générale

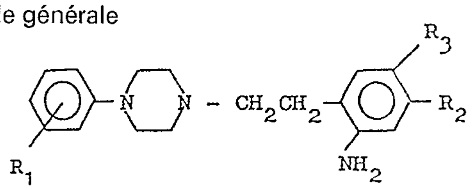

dans laquelle $R_1$, $R_2$ et $R_3$ sont définis comme dans la revendication 1, un aldéhyde de formule générale $R_4-CHO$ dans laquelle $R_4$ est défini comme dans la revendication 1 et un solvant tel que le benzène, en ce que l'eau formée au cours de la réaction est éliminée à l'aide d'un piège de Dean-Stark et en ce que le résidu est recristallisé dans un solvant tel qu'un mélange éther éthylique, éther de pétrole.

5. Composition pharmaceutique ou vétérinaire, caractérisée en ce qu'elle contient à titre de principe actif au moins un produit selon les revendications 1 à 3 en association avec un véhicule pharmaceutique ou un excipient approprié.

## Patentansprüche

1. Produkte gemäss der allgemeinen Formel

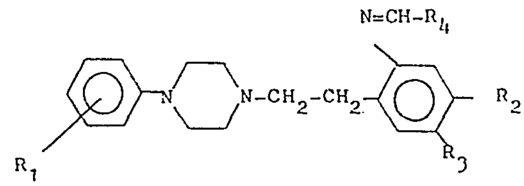

sowie nichttoxische Additionssalze derselben, in der $R_1$ H oder einen Substituenten oder mehrere in ortho-, meta- oder para-Stellung, ausgewählt unter $CH_3$, $CF_3$, Cl, $OCH_3$ und F; $R_2$ und $R_3$, die gleich oder verschieden sein können, H oder niederes Alkoxy mit $C_1$-$C_4$, wobei $R_2$ und $R_3$ zusammen eine Kette $-O-(CH_2)_n-O-$ mit n=1 oder 2 oder eine Kette $-O-CH_2-O-CH_2-$ bilden können; sowie $R_4$ Phenyl, mit einem Substituenten oder mehreren, ausgewählt unter Halogen, Alkyl mit $C_1$-$C_4$, Alkoxy mit $C_1$-$C_4$ und Trifluormethyl substituiertes Phenyl, Furyl oder Thienyl, unsubstituiert oder substituiert mit Halogen oder $CH_3$, oder Pyridinyl-2, -3 oder -4 bedeuten.

2. Produkte nach Anspruch 1, dadurch gekennzeichnet, dass $R_2$ und $R_3$=H bedeuten.

3. Produkte nach Anspruch 1, dadurch gekennzeichnet, dass $R_2$ und $R_3$ zusammen eine Kette $-O-CH_2-O-$ bilden.

4. Verfahren zur Herstellung der Produkte nach einem der Ansprüche 1-3, dadurch gekennzeichnet, dass man ein Gemisch aus einem Amin der allgemeinen Formel

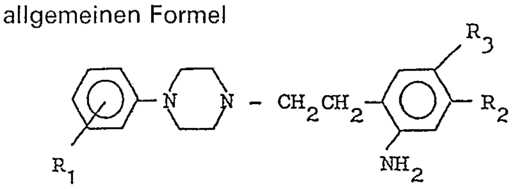

in der $R_1$, $R_2$ und $R_3$ wie in Anspruch 1 definiert sind, einem Aldehyd der allgemeinen Formel $R_4-CHO$, in der $R_4$ wie in Anspruch 1 definiert ist und einem Lösungsmittel, z.B. Benzol, erhitzt, das im Verlauf der Reaktion gebildete Wasser mit Hilfe einer Dean-Stark-Falle entfernt und den Rückstand aus einem Lösungsmittel, z.B. einem Gemisch aus Ethylether und Petrolether, umkristallisiert.

5. Pharmazeutische oder veterinärmedizinische Zusammensetzung, dadurch gekennzeichnet, dass sie als Wirkstoff mindestens ein Produkt nach einem der Ansprüche 1-3 zusammen mit einem pharmazeutischen Träger oder einem geeigneten Exzipienten enthält.

## Claims

1. Products corresponding to the general formula:

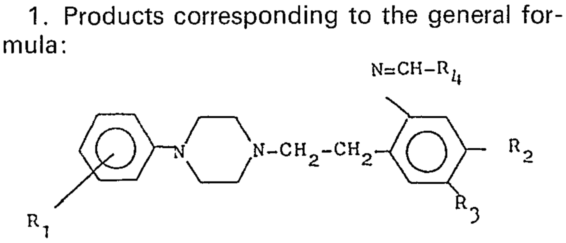

as well as the non-toxic addition salts thereof, in which $R_1$ stands for H or one or a plurality of ortho-, meta- or para-located substituents, selected among $CH_3$, $CF_3$, Cl, $OCH_3$ and F; $R_2$, $R_3$, identical or different, are H or a lower alcoxy from C1 to C4, $R_2$ and $R_3$ may together comprise a $-O-(CH)_n-O-$ chain with n=1 or 2, or a $-O-CH_2-O-CH_2-$ chain; $R_4$ stands for phenyl or phenyl substituted with one or a plurality of substituents selected among halogeno-, alkyl from C1 to C4, alcoxy from C1 to C4 and trifluoro-methyl, or $R_4$ is furyle or thienyl substituted with halogen or $CH_3$, or $R_4$ is 2-, 3- or 4-pyridinyl.

2. Products according to claim 2, characterized in that $R_2=R_3=H$.

3. Products according to claim 1, characterized in that $R_2$ and $R_3$ form together a $-O-CH_2-O-$ chain.

4. Method for preparing products according to claims 1 to 3, characterized in that one heats a mixture comprised of an amine with the general formula:

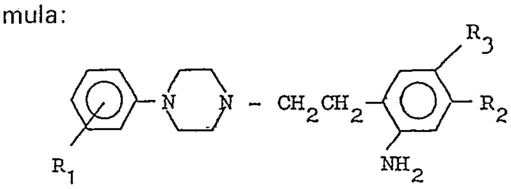

in which $R_1$, $R_2$ and $R_3$ are as defined in claim 1, an aldehyde with the general formula $R_4-CHO$, in which $R_4$ is as defined in claim 1, and a solvent such as benzene, in that the water formed during the reaction is removed by means of a Dean Stark's trap, and in that the residue is recrystallized in a solvent such as a mixture of ethyl ether, petroleum ether.

5. Pharmaceutic or veterinary composition, characterized in that it does contain as active constituent, at least one product according to claims 1 to 3 associated with a pharmaceutic medium or a suitable excipient.